(19) Europäisches Patentamt
European Patent Office
Office européen des brevets

(11) Veröffentlichungsnummer: **0 200 917**
**B1**

# (12) EUROPÄISCHE PATENTSCHRIFT

(45) Veröffentlichungstag der Patentschrift: **03.08.88**

(51) Int. Cl.⁴: **C 07 C 91/04**, C 07 C 89/00

(21) Anmeldenummer: **86104401.4**

(22) Anmeldetag: **01.04.86**

(54) **Di-(tert.-butanol)-amin und Verfahren zu seiner Herstellung durch Hydrierung von 2(1-Hydroxy-1-methyl-ethyl)-5,5-dimethyl-1,3-Oxazolin.**

(30) Priorität: **04.05.85 DE 3516028**

(43) Veröffentlichungstag der Anmeldung: **12.11.86 Patentblatt 86/46**

(45) Bekanntmachung des Hinweises auf die Patenterteilung: **03.08.88 Patentblatt 88/31**

(84) Benannte Vertragsstaaten: **CH DE FR GB LI NL**

(56) Entgegenhaltungen:
**FR - A - 2 328 701**

**JOURNAL OF THE CHEMICAL SOCIETY, PERKIN TRANSACTIONS II, 1983, Seiten 773-776; C. BONNINGUE et al.: "Bicyclic phosphanes. Part 2.1 Spontaneous dimerization of a constrained bicyclic phosphane. The crystal and molecular structure of a tricyclic compound containing the dioxadiazadiphosphecine ring"**
**CHEMICAL ABSTRACTS, Band 77, Nr. 10, 4. September 1972, Seite 71, Nr. 63302k, Columbus, Ohio, US; & JP - A - 71 34 957 (JAPAN SODA CO., LTD.) 13.10.1971**
**JOURNAL OF THE ORGANIC CHEMISTRY, Band 28, Nr. 4, April 1963, Seiten 1032-1034; J.R. GAINES et al.: "Reactions of alpha-hydroxy ketones with ammonia"**

(73) Patentinhaber: **HÜLS AKTIENGESELLSCHAFT, - RSP Patente / PB 15 - Postfach 13 20, D-4370 Marl 1 (DE)**

(72) Erfinder: **Brockhaus, Rudolf, Dr., Holsteiner Strasse 19, D-4370 Marl (DE)**
Erfinder: **Franke, Hans-Jürgen, Freiheitstrasse 13, D-4270 Dorsten (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Di-(tert.-butanol)-amin ist ein wertvolles Produkt, geeignet als Absorptionsmittel für saure Gase oder als Zwischenprodukt für zahlreiche Synthesen. Ähnliche Amine, z.B. Di-ethanolamin oder N-Di-isopropanolamin sind bereits als Absorptionsmittel bekannt; Di-(tert.-butanol)-amin hat den Vorteil, dass die OH-Gruppen als tertiäre Alkoholgruppen nicht oxydierbar sind. Darüber hinaus bietet das Amin zahlreiche Synthesemöglichkeiten am Stickstoff oder an den OH-Gruppen.

Das Di-(tert.-butanol)-amin kann durch Umsetzung von 2,2-Dimethyloxiran mit einem Überschuss an Ammoniak erhalten werden (Journal of the Chemical Society, Perkin Transaktions II, 1983, Seite 776). Diese Reaktion ist jedoch wenig selektiv.

Die Synthese von Di-(tert.-butanol)-amin gelingt überraschenderweise beim Hydrieren von 2(1-Hydroxy-1-methyl-ethyl)-5,5-dimethyl-1,3-Oxazolin mit Wasserstoff unter Hydrierbedingungen.

Dieses Oxazolinderivat ist leicht zugänglich, indem man alpha-Hydroxyisobutyraldehyd oder dessen inneres Acetal, ein Epoxid, mit Ammoniak bzw. wässrigem Ammoniak umsetzt.

Bei der Hydrierung dieses Oxazolinderivates unter relativ milden Bedingungen in Gegenwart von handelsüblichen Hydrierkatalysatoren, wie z.B. Raney-Nickel, Kupferchromit, Palladium- oder Platin-Kontakte, gegebenenfalls auf Trägern, z.B. auf Kohle, insbesondere A-Kohle, wie z.B. Pd-Kohle, öffnet sich der Ring und es entsteht das symmetrische Amin nach folgendem Schema:

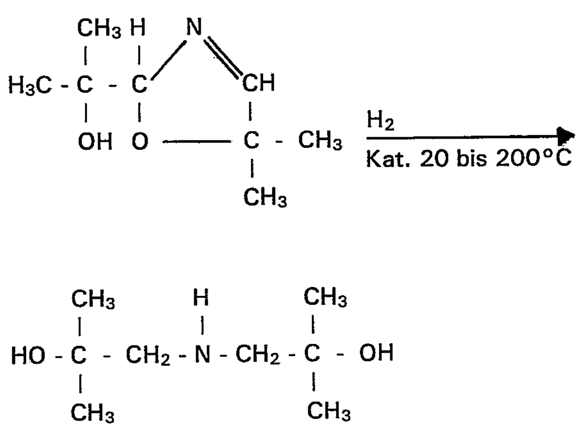


Die Hydrierung kann in Lösemitteln durchgeführt werden, z.B. in Methanol oder homologen Alkoholen. Die Temperatur und der Wasserstoffdruck können in weiten Grenzen schwanken. Im allgemeinen erfolgt die Hydrierung bei Temperaturen von 20 bis 200°C, bevorzugt bei 50 bis 150°C bei einem Druck bis 300 bar, vorzugsweise 30 bis 200 bar. Tieferer Wasserstoffdruck oder tiefere Temperaturen erfordern längere Reaktionszeiten. Diese Abhängigkeiten sind dem Fachmann bekannt. Steigert man die Temperatur über 200°C, so treten unerwünschte Nebenreaktionen auf. Zum Beispiel fällt α-Hydroxyisobuttersäureisobutylamid an oder unregelmässig kondensierte Amine.

Man kann die Hydrierung ein- und zweistufig durchführen. Bei der Hydrierung in zwei Stufen hydriert man bevorzugt in der zweiten Stufe bei höherer Temperatur und höherem Druck.

Die Reaktion lässt sich kontinuierlich und diskontinuierlich durchführen. Folgende Versuche zeigen beispielhaft die glatte Bildungsweise von Di-(tert.-butanol)-amin in Gegenwart von mehreren handelsüblichen Hydrierkatalysatoren unter den bei Hydrierung üblichen Bedingungen.

*Beispiel 1*

Herstellung von Di-(tert.-butanol)-amin durch Hydrieren des 2(1-Hydroxy-1-methyl-ethyl)-5,5-dimethyl-1,3-Oxazolins

In einen 1-l-Autoklav legt man 300 g 2(1-Hydroxy-1-methyl-ethyl)-5,5-dimethyl-1,3-Oxazolin (95,6%), 100 g Methanol und 20 g Raney-Nickel ($H_2O$-feucht) vor. Nach Entfernung der überstehenden Luft wird im Wasserstoff bis auf 100 bar aufgedrückt und gleichzeitig auf 50°C erwärmt. Der Ansatz wird unter diesen Bedingungen 13 Stunden gehalten, wobei der sich umsetzende Wasserstoff periodisch ersetzt wird. Anschliessend wird der Ansatz noch zwei Stunden bei 100°C und 150 bar Wasserstoffdruck gehalten.

Der Reaktionsaustrag (414 g) enthält lt. GC:

23,40 Gew.-% Methanol
5,10 Gew.-% 2(1-Hydroxy-1-methyl-ethyl)-5,5-dimethyl-1,3-Oxazolin
65,42 Gew.-% Di-(tert.-butanol)-amin

Hieraus errechnet sich ein Oxazolin-Umsatz von 92,4% und eine Di-(tert.-butanol)-amin-Ausbeute von 99,4%.

Das Amin destilliert über eine 30-cm-Kolonne bei einem Druck von 25 mbar bei ca. 126°C. Die Reinheit liegt bei = 99% lt. GC und Titration.

Das Amin ist farblos und in Wasser, Methanol und Aceton gut löslich. Sein Schmelzpunkt beträgt 31 bis 32°C.

*Beispiele 2 bis 8*

In diesen Beispielen werden Katalysator, $H_2$-Druck, Temperatur und Zeit von Beispiel 1 variiert. Die Bedingungen und Resultate sind tabellarisch aufgeführt.

*Beispiele*

Hydrierung von 2(1-Hydroxy-1-methyl-ethyl)-5,5-dimethyl-1,3-Oxazolin
Ansatz, Ausrüstung etc., wie im Beispiel 1 beschrieben

| Beispiel | Dauer (h) Stufen I | Dauer (h) Stufen II | Druck bar (atm) Stufen I | Druck bar (atm) Stufen II | Temperatur (°C) Stufen I | Temperatur (°C) Stufen II | Katalysator | Umsatz (%) | Selektivität (%) |
|---|---|---|---|---|---|---|---|---|---|
| 1 | 13 | 2,0 | 100 | 150 | 50 | 100 | Ra-Ni | 92,6 | 99,4 |
| 2 | 6 | 35,0 | 100 | 200 | 100 | 150 | Ra-Ni | 99,0 | 74,0+20% Amid [1] |
| 3 | 3 | 4,5 | 100 | 200 | 150 | 200 | Ra-Ni | 97,9 | 65,0+ 8% Amid[1] |
| 4 | 24 | — | 200 | — | 70 | — | Ra-Ni | 68,2 | 99,1 |
| 5 | 24 | — | 200 | — | 180 | — | Ra-Ni | 98,1 | 10,0 |
| 6 | 24 | — | 200 | — | 104 | — | 5% Pd auf A-Kohle | 99,0 | 98,3 |
| 7 | 15 | — | 30 | — | 107 | — | 5% Pd auf A-Kohle | 67,8 | 99,2 |
| 8 | 6 | — | 200 | — | 150 | — | Cu-Chromit (nach Adkins) | 99,4 | 98,4 |

[1] α-Hydroxyisobuttersäureisobutylamid

**Patentansprüche**

1. Verfahren zur Herstellung von Di-(tert.-butanol)-amin, dadurch gekennzeichnet, dass man 2(1-Hydroxy-1-methyl-ethyl)-5,5-dimethyl-3-Oxazolin in Gegenwart von Hydrierkatalysatoren bei einem $H_2$-Druck bis 300 bar und Temperaturen von 20 bis 200°C hydriert.

2. Verfahren nach Anspruch 1, dadurch gekennzeichnet, dass man die Hydrierung bei 50 bis 150°C durchführt.

3. Verfahren nach den Ansprüchen 1 und 2, dadurch gekennzeichnet, dass man als Hydrierkatalysatoren Raney-Nickel, Kupferchromit, Palladium- oder Platin-Kontakte, gegebenenfalls auf Trägern, einsetzt.

4. Verfahren nach den Ansprüchen 1 bis 3, dadurch gekennzeichnet, dass man die Hydrierung bei 30 bis 200 bar durchführt.

**Claims**

1. A process for the preparation of di-(tert.-butanol)-amin, characterized in that 2(1-hydroxy-1-methyl-ethyl)-5,5-dimethyl-3-xazoline is hydrogenated in the presence of hydrogenation catalyst(s) at an $H_2$ pressure of up to 300 bar and at temperatures of 20 to 200°C.

2. A process according to claim 1, characterized in that the hydrogenation is carried out at 50 to 150°C.

3. A process according to claim 1 or 2, characterized in that Raney nickel, copper chromite or palladium or platinum contact catalyst(s), optionally on carrier(s), are employed as hydrogenation catalyst.

4. A process according to any of claims 1 to 3, characterized in that the hydrogenation is carried out at 30 to 200 bar.

**Revendications**

1. Procédé de préparation de di-(ter.-butanol)-amine, caractérisé par le fait que l'on hydrogène de la 2-(1-hydroxy-1-méthyl-ethyl)-5,5-diméthyl-3-oxazoline en présence de catalyseurs d'hydrogénation sous une pression de $H_2$ en allant jusqu'à 300 bars et à des températures de 20 à 200°C.

2. Procédé selon la revendication 1, caractérisé par le fait que l'on effectue l'hydrogénation à une température de 50 à 150°C.

3. Procédé selon les revendications 1 et 2, caractérisé par le fait que l'on utilise, comme catalyseurs d'hydrogénation, du nickel Raney, de la chromite de cuivre, des catalyseurs au palladium ou au platine, éventuellement sur des supports.

4. Procédé selon les revendications 1 à 3, caractérisé par le fait que l'on effectue l'hydrogénation sous une pression de 30 à 200 bars.